# EUROPEAN PATENT APPLICATION

(11) **EP 3 640 949 A1**
(43) Date of publication of application: **22.04.2020**
(21) Application number: 18200983.7
(22) Date of filing: 17.10.2018
(51) Int. Cl.: G16H 30/40, G16H 30/20, G16H 40/67

(54) **AUGMENTED REALITY WITH MEDICAL IMAGING**

(71) Applicant: Incremed AG, 8008 Zurich (CH)
(72) Inventor: BAY, Till, 8004 Zürich (CH); KRAUTZ, Christoph, 8044 Zürich (CH); GULL, Judith, 8045 Zürich (CH); PETER, Ueli, 8037 Zürich (CH); HERTIG, Samuel, 8049 Zürich (CH); FARSHAD, Mazda, 8126 Zumikon (CH)
(74) Representative: Ko, Benjamin Hyensuk

(57) **Abstract**

An augmented reality system for displaying a medical image (in particular an image stream) and a respective method are proposed. The augmented reality system comprises an optical head mounted display (1), a medical imaging device (2), a tracking system (3) that is adapted to measure data concerning a position and orientation of at least one object, and a processing unit (4). The processing unit is configured to transform an image from a coordinate system in which the image was taken to a coordinate system of the display (1). This transformation is performed using data measured by the tracking system (3), where the ARS is adapted to display the transformed image on the display (1) in a position, orientation and scale that corresponds to the perspective of a position and orientation of the bearer of the display (1).

## Description

### TECHNICAL FIELD

The present invention relates to the field of medical imaging.

### PRIOR ART

Medical images are used in state-of-the-art medical procedures to provide computer-aided guidance and increase accuracy and precision during an intervention. For example, for a brachial plexus block, the operator typically holds an ultrasound device in one hand and a syringe in the other, so that they can see the needle of the syringe in the ultrasound image. Current state-of-the-art procedures usually involve displaying the ultrasound image on a screen that is located next to the patient, which results in the operator effectively looking away from the position at which they are inserting the syringe. Therefore, this kind of operation is counter-intuitive and requires a lot of training to perform safely.

In recent years, optical head mounted displays ("OHMD") have become available, which a bearer can wear on his head and which comprise a display that is arranged in the field of view of the bearer. Such an OHMD is adapted so that the bearer can see at least a part of his real environment as well as computer-generated images shown in the field of view of the bearer. This allows the bearer to visually experience an augmented reality, part of which is real and part of which is computer-generated. The computer-generated images can be displayed to simulate two- and three-dimensional objects that are shown as holograms overlaid onto the real world.

### SUMMARY OF THE INVENTION

The purpose of the invention is to provide an improved method and system for utilizing medical imaging. Further features of the invention are laid down in the dependent claims.

The present invention concerns creating an augmented reality ("AR"), i.e. a perception of a real-world environment combined with computer-generated information, by displaying medical images in the field of view of a user.

An AR system ("ARS") is proposed that is adapted for displaying a medical image and comprises
- an OHMD that is designed to display images;
- a medical imaging device ("MID") that is designed to take, preferably in-situ, an image (i.e. the medical image);
- a tracking system ("TS") that is adapted to measure data concerning a position and orientation of at least one object, preferably at least one object (e.g. the MID and/or the OHMD); and
- a processing unit that is configured to transform an image taken by the MID from a first coordinate system to a second coordinate system using data measured by the TS,
wherein the ARS is adapted to display the transformed image on the OHMD in a position, orientation and scale that corresponds to the perspective of a position and orientation of the OHMD ("PO_{OHMD}"). The ARS can comprise hardware means and software means.

In other words, the ARS is adapted so that
- an image (preferably a series of images, e.g. an image stream/video) can be taken by the MID,
- the image can be transformed using data measured by the TS, and
- the transformed image can be displayed to the bearer, thereby creating the impression that the image is displayed from the perspective of the current view of the OHMD.

This can allow for creating the AR impression that a part of reality at which the image was taken is overlaid with the transformed image.

The image is transformed so that the bearer of the OHMD, i.e. from the perspective of the PO_{OHMD}, perceives the transformed image as being in the position and orientation of the image ("PO_{image}"). To do this, the image, which is initially created according to a coordinate system of the image ("CS_{image}"), is transformed to a coordinate system ("CS_{OHMD}") that is chosen to match the view of the bearer.

Overlaying parts of the reality with a medical image that is adjusted to the viewers perspective can support the work of an operator by allowing him to look in the direction of the area at which he performs his tasks, thereby allowing him to work in a more intuitive manner. Using the proposed ARS can thus allow for greatly reducing the time and costs for training of medical staff. In contrast to so-called virtual reality settings, where the real world is completely obscured to the bearer of a head mounted display device, the OHMD of the proposed ARS allows the bearer to still see the real world, which is preferred for medical procedures.

Currently used systems comprise a monitor and a supporting stand on which the monitor is placed. Therefore, currently used systems are often bulky and do not allow for a disinfection in a convenient and speedy manner. Thus, these systems are often not used in operating rooms, which are often tightly packed and require sterility. Since the proposed ARS can be realized in a significantly smaller and/or space efficient manner than the currently used systems, it can allow for using medical imaging in scenarios in which the currently used systems cannot conveniently be used, e.g. for cross-checking the state of an ongoing intervention in a tightly packed operating room. The possible decrease in size can furthermore allow the proposed ARS to be realized in an easily transportable manner, thereby facilitating its use in rural areas, mobile or field hospitals, and/or in clinics or hospitals lacking state-of-the-art infrastructure.

The proposed ARS and the proposed method can be used to support diagnostic and therapeutic operations. They can for example be used in the context of
- joint infiltration,
- interscalene block / regional anesthetic,
- perineural anesthesia,
- treatment of enthesitis,
- paracentesis, and/or
- resection.

For example, if during a resection an operator intends to remove parts of a tissue, structure, or organ of the patient's body that they can distinguish using a medical imaging method, they can use the proposed ARS during the intervention to determine which parts they shall remove and/or to determine which parts they have already removed.

Methods for taking the medical image by the MID can for example include
- X-Ray imaging (e.g. X-Ray computer tomography),
- magnetic resonance imaging,
- ultrasound imaging,
- thermal imaging and/or
- nuclear medicine imaging (e.g. single-photon emission computed tomography or positron emission tomography).

In some embodiments, the medical imaging method allows for taking two-dimensional ("2D") and/or three-dimensional ("3D") images. For example, X-ray technology typically allows for 2D-images and multiple such X-ray images allow for computing a 3D computer tomographic image ("CAT scan"). Another example of a 3D-imaging method is 3D-ultrasound imaging.

In some embodiments, the MID comprises an ultrasound probe. The ultrasound probe can e.g. be comprised in a handheld device.

In some embodiments, the MID comprises an X-ray emitter and an X-ray plate, which e.g. can be comprised in a C-arm.

For some medical imaging methods, the image does not have an inherent position, orientation and/or scale. Therefore, the position, orientation and/or scale of the image should be understood to be a choice, preferably a choice that approximates the intuitive understanding of the image, e.g. matching the anatomy of the respective body part. For example, an X-ray image is a two dimensional projection of a three dimensional segment. It can allow for allocating a scale (e.g. the size in which it was taken) and an orientation (e.g. orthogonal to the X-ray plate), but not for allocating an unambiguous position, as the X-ray image represents a superimposition of all the planes that the X-rays have passed through. Therefore, the ARS can be adapted to make a choice, e.g. displaying the X-ray image as being at the position of the X-ray plate or displaying the X-ray image as being somewhere between the X-ray emitter and the X-ray plate, e.g. so that the image is displayed as being halfway in the body part that has been scanned.

In some embodiments, the ARS comprises adjusting means that is adapted to instruct the ARS to adjust the position, orientation and/or scale in which the transformed image is displayed. The adjusting means can allow the bearer to instruct the ARS to not display the transformed image corresponding to the currently displayed perspective of a position and orientation of the image, but somewhere else and/or somehow else, e.g. scaled to a different size.

In some embodiments, the adjusting means are adapted so that the user can adjust the choice made by the ARS with respect to a position, orientation and/or scale of the image in cases where at least one thereof cannot be allocated in an unambiguous way. In a variant, the ARS is configured to be able to remember adjustments to the choices.

In some embodiments, the adjusting means is adapted to instruct the ARS to display
- a (larger or smaller) scaling factor ("zoom") of the transformed image or parts thereof;
- a rotation of the transformed image; and/or
- the transformed image at a side of the display.

An adjusted view can support the operator during an intervention, e.g. when the actual size of the image generated by the MID is too small for the operator to see all the details necessary for the success of the medical intervention.

In some embodiments, the medical imaging method allows for taking still images (i.e. single images) and/or for taking a series of images. Preferably, the ARS is adapted to take a series of images quasi continuously, e.g. at a rate of 10 images or more per second, to create the illusion of a continuously changing image such like an image stream/video. In some embodiments, the ARS is adapted to take a series of 3D images, such as a 3D image stream/video, e.g. using 3D ultrasound. Still images can be preferred if taking an image is expensive or bears health risks, such as taking an X-ray.

In some embodiments, the ARS is adapted to display the transformed image in quasi-real-time, e.g. in less than 100 milliseconds, preferably in less than 60 or 40 milliseconds, after the image was taken and/or the PO_{OHMD} has changed. For example, where the image is taken quasi continuously, the PO_{OHMD}-adjusted transformation of an image is displayed with a delay of less than 100 milliseconds after the image was taken. In another example, the image is a still image, e.g. an X-ray image, which is adjusted to a new PO_{OHMD} with a delay of less than 100 milliseconds. A delay of less than 100 milliseconds can create an illusion of real-time observation, which can allow for a more intuitive usage of the ARS.

In some embodiments, the ARS is configured so that the transformed image of an image taken at a distance of 2 m or less from the OHMD is displayed with a (perceived) precision of 5 mm or less, preferably 3 mm or less or 1 mm or less; in other words so that a pixel of the transformed image is not displayed as being further away from a position of the reality that is intended to represent than a further position of reality that in reality is less than 5 mm, preferably 3 mm or less or 1 mm or less, away from said position.

In some embodiments, the MID comprises, preferably is, a freely movable device, such as a handheld device, a semi-movable device, such as a C-arm, or a static device. For example, an ultrasound device can comprise a handheld device that comprises the ultrasound sensor so that the position and orientation of the image can easily be altered by moving the handheld device. In another example an X-ray device can comprise a C-arm, whose mobility is limited.

In some embodiments, the display of the OHMD comprises two portions, each of which is adapted to be arranged in front of one of the eyes of the bearer. The OHMD can be adapted for stereo displaying, i.e. creating the illusion of a 3D image to its bearer.

In some embodiments, the OHMD is adapted for a near focus of the bearer's view, e.g. such that it is suitable for medical procedures carried out at arm's length, preferably the OHMD is adapted for focusing on a distance of 20 cm to 100 cm.

Data measured by a TS can allow determining the position and orientation of an object, which in turn can allow for determining the transformation from a coordinate system of said object to a coordinate system of a different object or vice versa. Said object can e.g. be the MID or the OHMD.

By measuring the data of the TS over time, the ARS can track the position and orientation of an object and thus can allow for adjusting the transformation according to the current position and orientation of an object, preferably in quasi-real-time. The ARS, e.g. the TS, can be adapted to track the position and orientation of two or more objects, e.g. the MID and the OHMD.

The measuring means of the TS can e.g. comprise optical means (i.e. using electromagnetic radiation in the visible-light spectrum), infrared means (i.e. using electromagnetic radiation in the infrared spectrum), magnetic means (i.e. using magnetic fields), and/or image and shape recognition means (i.e. using image recognition and shape recognition). The TS can comprise an inertial measurement unit ("IMU") that is adapted to measure data concerning a spatial acceleration and a rotation rate.

In some embodiments, the ARS comprises two or more TSs, which preferably are part of a redundant system. The redundant system can e.g. allow to increase the precision of measurements resp. calculations using the measurements (e.g. by taking averages and/or applying a Kalman filter), increase the reliability of the ARS (e.g. by performing independent measurements) and/or collect calibration data. Preferably, at least two of the two or more TSs comprise different sensor technology (e.g. a first TS using infrared recognition of a marker and a second TS using shape recognition) and/or different sensor configurations (e.g. a first TS being fixedly attached to the OHMD and a second TS being not fixedly attached to the OHMD). The ARS can be adapted to use a Kalman filter to calculate the current positions and orientations of at least some of the tracked objects by fusing data measured by two or more TSs. Preferably, the ARS can be adapted to use a Kalman filter to calculate resp. estimate the current positions and orientations of at least some of the tracked objects by taking into account earlier calculated positions and orientations of the tracked objects, e.g. in cases where one or more TSs of the two or more TSs fail to supply reliable data.

Preferably, a coordinate system of an object, e.g. the image, the MID, the TS or the OHMD, is a coordinate system that is associated with said object in the sense that the position, and preferably the orientation, of said object of fixed with respect to said coordinate system. Typically, there are multiple choices for such a coordinate system. Preferably, a coordinate system is chosen, wherein the origin, and possibly the direction of axes, are adjusted to the object in question, e.g. where the origin is placed at the location of a sensor of said object. Preferably, the coordinate system is a Cartesian coordinate system, which in three dimensions has three axes that are perpendicular to one another. Other possible choices include polar, cylindrical or spherical coordinate systems.

In some embodiments, the transformation comprises steps represented by matrices, e.g. expressing a translation, a scaling and/or a rotation. The matrices can e.g. be 4x4 matrices, preferably whereby a 3x3 block represents a scaling and a rotation and a 3x1 block (vector) represents a translation. The remaining entries can e.g. be chosen to guarantee that the matrix is invertible. The composition of steps of the transformation represented by matrices can be represented by the multiplication of said matrices. Using matrices and their multiplication can allow for a quick calculation of the transformed image, which in turn can allow for a quasi-real-time adjustment of the image displayed on the OHMD to the current PO_{OHMD} and, if a sequence of images is taken, to the current image.

The transformation can further comprise perspective transformations that allow for displaying an image on the OHMD in a perspective manner. The transformation can comprise stereo transformations that allow for stereo-displaying.

In some embodiments, the first coordinate system is the CS_{image} or a CS_{MID} and the second coordinate system is a CD_{MID} or the CS_{OHMD}. For example, the processing unit can be configured to transform the image from the CS_{image} to a coordinate system of the MID that took the image ("CS_{MID}") and then transform the image from this CS_{MID} to the CS_{OHMD}.

In some embodiments, the PO_{image} is fixed relative to the position and orientation of the MID ("PO_{MID}") and a transformation from the CS_{image} to the CS_{MID} can be a constant transformation. For example, the transformation of the CS_{image} to the CS_{MID} is trivial if the image is directly taken in the CS_{MID} (so that the CS_{image} is identical to the CS_{MID}).

In some embodiments, the PO_{image} is not fixed relative to PO_{MID}, i.e. the MID can, without itself being moved, take images at different position or orientation. For example, a sensor of the MID can be movable or the MID can comprise adequate software means. In this case it can be possible to determine the transformation of the CS_{image} to the CS_{MID} using data of the MID (e.g. data of the control means of the MID) in combination with calibration data.

In some embodiments, the ARS, preferably the TS, is adapted to measure data concerning the PO_{OHMD} relative to the PO_{image} resp. the PO_{MID}. The ARS can be adapted to track the PO_{OHMD} relative to the PO_{image} resp. the PO_{MID}. Preferably, the ARS is adapted to transform the image from the CS_{image} to the CS_{OHMD} using data concerning the PO_{OHMD} relative to the PO_{image} resp. the PO_{MID}. In some cases, e.g. where a still image is displayed, the PO_{image} is constant and the ARS can be adapted to measure the PO_{OHMD} relative to this constant position and orientation, i.e. relative to a world reference.

In a preferred embodiment, the ARS is adapted for displaying a medical image in quasi-real-time and comprises
- the OHMD that is designed to display images;
- the MID that is designed to take an image (i.e. the medical image);
- the first TS that is adapted to measure data concerning a position and orientation of at least one object of the ARS (e.g. the MID and/or the OHMD); and
- the processing unit that is configured to transform an image taken by the MID from the CS_{image} to the CS_{OHMD} using data measured by the TS,
wherein the ARS is adapted to display the transformed image in quasi-real-time on the OHMD in a position, orientation and scale that corresponds to the perspective of the PO_{OHMD}.

In some embodiments, the processing unit is configured
- to transform the image taken by the MID from the CS_{image} to a CS_{MID},
   o e.g. by using data concerning the position and orientation of a sensor of the MID relative to the PO_{MID};
- to transform said image from said CS_{MID} to a CS_{TS} using data measured by the TS,
   o e.g. by using data concerning the PO_{MID} measured by the TS; and
- to transform said image from said CS_{TS} to the CS_{OHMD},
   o e.g. by using data concerning the PO_{OHMD} measured by the TS,
wherein CS_{TS} denotes a chosen coordinate system of the TS. The transformation from CS_{image} to CS_{MID} can be a constant transformation if the position and orientation of the sensor of the MID is fixed relative to the PO_{MID}. The transformation from CS_{TS} to CS_{OHMD} can be a constant transformation if the PO_{OHMD} is fixed relative to the TS.

In some embodiments, the PO_{OHMD} is fixed relative to the TS. In other words, a position and orientation relative to which the TS is adapted to measure is fixed relative to the PO_{OHMD}. This means that a chosen CS_{TS} can be transformed to the CS_{OHMD} using a constant transformation.

For example, the TS can comprise a radiation (e.g. optical or infrared) emitter and a radiation receiver, whereof at least the receiver is fixedly attached to, preferably integrated into, the OHMD. Fixedly attached means that any change to the position or orientation of the one object will inevitably lead to the same change to the position (i.e. same translation) and orientation (i.e. same rotation) of the other object to which it is fixedly attached. However, it may of course still be the case that the two objects can again be separated, e.g. where they are fixedly attached by removable screws. A transformation of a first coordinate system of a first object that is fixedly attached to a second object to a second coordinate system of the second object can be a constant transformation. In the example at hand, the transformation from the CS_{TS} to the CS_{OHMD} can be a constant transformation and thus independent of the PO_{OHMD}.

In some embodiments, the PO_{OHMD} is not fixed relative to the TS. For example, the TS can be a static system, which e.g. is attached to a wall or mounted on a stand that is placed in a room. In this case, the calculation of the transformation from the CS_{image} or the CS_{MID} to the CS_{OHMD} can be performed using data concerning the PO_{OHMD}, wherein said data are preferably measured using the TS.

In some embodiments, the PO_{OHMD} is not fixed relative to the TS. Preferably, the TS is adapted to measure data concerning the PO_{MID} and the PO_{OHMD}. The processing unit can be configured to transform the image from a CS_{MID} to the CS_{OHMD} by using the measured data concerning the PO_{MID} and by using the measured data concerning the PO_{OHMD}, e.g. by
- transforming the image from a CS_{image} to a CS_{MID}, and then
- transforming the image from the CS_{MID} to a CS_{TS} by using the measured data concerning the PO_{MID}, and then
- transforming the image from the CS_{TS} to the CS_{OHMD} by using the measured data concerning the PO_{OHMD}.

In some embodiments, the ARS comprises a first TS and a second TS, wherein each is adapted to measure data concerning a position and orientation of at least one object of the ARS, e.g. the MID and/or the OHMD.

In some embodiments, the TS comprises a first TS and a second TS and the ARS is adapted to calculate the position and orientation of objects with increased precision, e.g. by fusing the data measured by the various TSs. For example, the processing unit can be adapted to calculate the position and orientation of an object (e.g. the PO_{MID} or the PO_{OHMD}) by taking weighted averages of the position and orientation of said object as calculated using data measured by the first TS and as calculated using data measured by the second TS and/or by using a Kalman filter to fuse the respective data.

In some embodiments, the ARS is adapted to collect and/or use calibration data. Calibration data can allow for correcting systematic deviations, which e.g. can be due to production tolerances. Preferably, calibration data are determined using data measured by a first TS and using data measured by a second TS.

In some embodiments, calibration data are used for determining transformations, e.g. the transformation from the CS_{image} or a CS_{MID} to the CS_{OHMD} can be determined using data concerning the PO_{MID}, and possibly data concerning PO_{OHMD}, as well as calibration data. For example, the transformation from CS_{image} to CS_{MID} can be calculated using calibration data, e.g. wherein PO_{image} is fixed relative to PO_{MID} and the transformation from CS_{image} to CS_{MID} is constant. In cases the PO_{image} is not fixed relative to the PO_{MID}, e.g. where the image sensor of the MID is movable relative to the rest of the MID, the transformation from CS_{image} to CS_{MID} may not be constant but can e.g. be calculated using calibration data in connection with information concerning the movement of a motion unit of the sensor.

In some embodiments, the ARS, preferably the processing unit of the ARS, is configured to transform the image taken by the MID from the CS_{image} to the CS_{OHMD}
- using data measured by the first TS and
- using calibration data that were determined using data measured by the first TS and data measured by the second TS.

Preferably, the calibration data are pre-determined and stored in the ARS.

In some embodiments, the calibration data are determined a using simultaneous measurement by the first TS and by second TS of the same environment. The calibration data can then e.g. be determined by solving equation systems concerning the respectively measured point cloud. In an example, the calibration data are determined using a simultaneous measurement concerning a position and orientation of the same object. Said same object could e.g. be a dummy object, which is specifically used for calibration purposes.

In some embodiments, the TS comprises a first TS and a second TS and the ARS is adapted to conduct plausibility checks, e.g. verifying data measured by the first TS using data measured by the second TS or vice versa. Preferably, the ARS can be configured to stop displaying the medical image and/or to issue a warning if the information of the data measured by the second TS significantly deviates from the information of the data measured by the first TS, e.g. in cases where data concerning the PO_{MID} as measured by the first TS are deemed inconsistent with data concerning the PO_{MID} as measured by the second TS.

In some embodiments, the TS comprises a first TS and a second TS and the ARS is adapted to measure data concerning a position and orientation of a first object using data measured using the first TS and to measure data concerning the a position and orientation of a second object using the second TS.

In some embodiments, the processing unit is configured to transform the image taken by the MID from the CS_{image} to the CS_{OHMD} according to multiple ways, e.g. a first way and a second way. The processing unit can be adapted to always calculate the transformation in multiple ways or to only do so on specific occasions.

In some embodiments, the ARS comprises a first TS and a second TS and is adapted to transform the image taken by the MID from the CS_{image} to the CS_{OHMD} according to a first way and according to a second way,
- wherein the first way comprises transforming the image taken by the MID from the CS_{image} to the CS_{OHMD} using data measured by the first TS, and
- wherein the second way comprises transforming the image taken by the MID from the CS_{image} to the CS_{OHMD} using data measured by the second TS.

In some embodiments, the TS comprises a first TS and a second TS and the ARS is adapted to calculate the transformed image with increased precision, e.g. by fusing the data measured by the various TSs. For example, the processing unit can be adapted to calculate the transformed image by taking weighted averages of the transformed image as calculated according to a first way using data measured by the first TS and of the transformed image as calculated according to a second way using data measured by the second TS.

In some embodiments, at least one TS is fixedly attached to the MID. Preferably a first TS is not fixedly attached to the MID and a second TS is fixedly attached to the MID.

In some embodiments, the ARS, e.g. the second TS, comprises an inertial measurement unit ("IMU") that is adapted to measure data concerning a spatial acceleration and a rotation rate of an object, e.g. the MID or the OHMD. The IMU can be adapted to intrinsically measure data concerning a spatial acceleration and a rotation rate of an object, e.g. by being fixedly attached to said object, i.e. that any change of a position and orientation of said object will inevitably lead to the same change of a position (i.e. same translation) and orientation (i.e. same rotation) of the IMU.

In some embodiments, the ARS, e.g. the IMU, comprises an accelerometer that is adapted to measure data concerning a spatial acceleration of an object. The accelerometer can e.g. comprise piezo-electric, piezo-resistive and/or capacitive components. The accelerometer can e.g. comprise a pendulous integrating gyroscopic accelerometer.

In some embodiments, the ARS, e.g. the IMU, comprises a gyroscope that is adapted to measure data concerning a rotation rate of an object.

Using data concerning a spatial acceleration and rotation rate of an object, it is possible to determine a relative movement and rotation, i.e. a variation of the position and orientation of said object, and thus to estimate the position and orientation of said object at a time *t*, e.g. by using
- a known position and orientation of an object at a first time *t₀,* e.g. at a time *t₀*=*0*, (as e.g. measured by a, e.g. the first, TS) and
- the variation of the position and orientation of the object as calculated based on the data concerning a spatial acceleration and rotation rate since said first time *t₀*.

Thus, data measured by the IMU can be used for estimating the position and orientation of an object of the ARS, e.g. the MID and/or the OHMD. Preferably, the processing unit is configured to use a Kalman filter for calculating said estimates. For example, the processing unit can be configured to verify and/or correct data measured by a TS (e.g. the first TS) using the data of the IMU (which e.g. can be comprised in the second TS).

In some embodiments, the processing unit is adapted to calculate the transformation using a position and orientation of an object, e.g. the MID and/or the OHMD, and is further adapted to calculate the position and orientation of said object using data measured by an IMU that is fixedly attached to said object.

In some embodiments, the second TS comprises an IMU that is fixedly attached to the MID, e.g. to a detector thereof, and that is adapted to measure data concerning a spatial acceleration and a rotation rate of the MID. Preferably, the processing unit is adapted to calculate the relative movement and rotation of the MID using data measured by the IMU. The processing unit can be configured
- to estimate the current PO_{image} (resp. PO_{MID}) using
   o the data measured by the IMU and
   o an earlier determined PO_{image} (resp. PO_{MID}) calculated using data measured by the first TS; and
- to transform the image taken by the MID from the CS_{image} to the CS_{OHMD} using the estimated PO_{image} (resp. PO_{MID}).

Similarly, the processing unit can be adapted to calculate the transformation using an estimated position and orientation of another object (e.g. of the OHMD) if an IMU is fixedly attached to said other object.

In some embodiments, the processing unit is configured to transform the image taken by the MID from the CS_{image} to the CS_{OHMD}
- according to a first way using data measured by the first TS and
- -according to a second way using data measured by the first TS and by a second TS comprising an IMU.

Preferably, the first way comprises using data measured by the first TS at a current time t and the second way comprises data measured by the first TS at an earlier time *t₀* (*t>t₀*) and data measured by the second TS since the earlier time *t₀*. In an example, the current time t is less than 5 seconds later than the earlier time *t₀*.

In some embodiments, the TS comprises a first TS and a second TS and the ARS is adapted to calculate the transformation using the data measured by first TS in a first mode and using the data measured by the second TS in a second mode.

In some embodiments, the ARS comprises a first TS and a second TS, wherein the second TS preferably comprises an IMU. Preferably, the ARS is configured to normally calculate a transformation using data measured by the first TS (first mode) and, upon the occurrence of a triggering (e.g. if measurements by the TS are deemed unreliable), to calculate said transformation using data measured by the second TS (second mode). Preferably, the first TS and the second TS comprise different sensor technology and/or different sensor configurations, whereby the probability that both TSs are unreliable at the same time can be reduced.

In some embodiments, the ARS is configured
- to normally operate in a first mode in which the transformed image that is displayed is calculated according to a first way, and
- to switch to a second mode in which the transformed image that is displayed is calculated according to a second way upon the occurrence of a triggering event.
Preferably, the triggering event comprises that measurements on which the first way is based are deemed unreliable, e.g. with regard to accuracy or latency. The triggering event can e.g. occur if a plausibility test on measured data and/or on a calculated position and orientation has failed or where a calculation routine is unexpectedly terminated.

In some embodiments, the first TS is infrared-based, the second TS is based on the measurements of an IMU and the processing unit is configured to normally calculate the transformation of the CS_{image} to the CS_{OHMD} using a calculation of the PO_{MID} using data measured by the infrared based TS (first mode). The processing unit is further configured to - in case the measurement of the infrared based TS is deemed unreliable, e.g. when the infrared radiation is obstructed and thus the PO_{MID} can no longer be calculated - calculate the transformation of the CS_{image} to the CS_{OHMD} using an estimate of the PO_{MID} using data measured by the IMU-based TS (second mode), namely by estimating the current PO_{MID} using
- the last reliable PO_{MID} calculated based on data measured by the infrared-based TS and
- the relative movement and rotation calculated based on the measurements of the IMU-based TS.

In some embodiments, the ARS comprises monitoring means for determining if a measurement of the TS shall be deemed unreliable. The monitoring means can comprise hardware means and/or software means. Preferably, the monitoring means comprises a plausibility check, e.g. on the data measured by a, preferably at least the first, TS. The monitoring means can be adapted to indicate the occasion of a triggering event.

In some embodiments, the monitoring means comprises a second TS, preferably a second TS using a different sensor technology and/or a different sensor configuration. For example, the first TS can use a sensor system based on visible and/or infrared radiation, while the second TS can use a sensor system based on an IMU. In another example, the first TS is fixed relative to the OHMD and the second TS is not fixed relative to the OHMD (or vice versa).

In some embodiments, the ARS comprises a first TS whose measurements are used for a first mode, a second TS whose measurements are used for a second mode and a third TS whose measurement are used for a decision whether the first mode or the second mode shall be used. Preferably, at least the second TS comprises an IMU.

In some embodiments, the processing unit is configured to transform the image taken by the MID from a first coordinate system, e.g. the CS_{image}, to a second coordinate system, e.g. the CS_{OHMD} in a first way and in a second way. Preferably, the ARS is configured to switch from a first mode in which the transformation is calculated in the first way to a second mode in which the transformation is calculated in the second way. In an example, the first mode is normally used and the second mode is used if the measurements on which the first way is based are deemed unreliable. Preferably, the second way comprises the usage of data measured by an IMU.

In some embodiments, the ARS is adapted to track and display multiple images. For example, where X-ray image were taken of different parts of a patient's body, the ARS can be adapted to keep track of the respective image and display the transformation of the suitable image or images depending on the current PO_{OHMD}. In order to keep track of the various images and possibly the various MIDs, the ARS can use a common reference system.

In some embodiments, the ARS comprises a position and orientation marker ("POM"). POMs are used to support the tracking of objects of the ARS, e.g. the MID or the OHMD, using the TSs. Preferably, at least one TS, e.g. at least the first TS, of the ARS is adapted to measure data concerning a position and orientation of the POM ("PO_{POM}").

In some embodiments, the POM is fixedly attached to an object of the ARS, e.g. the MID or the OHMD. Fixed attachment means that any change to the position and orientation of said object will inevitably lead to the same change to the position (i.e. same translation) and orientation (i.e. same rotation) of the POM. Thus, the position and the orientation of the object can be calculated using data concerning the PO_{POM}, and possibly using calibration data, e.g. to eliminate production tolerances of the attachment of the POM to said object. Preferably, multiple POMs are fixedly attached to same object, e.g. to different sides of said object.

In some embodiments, the ARS comprises multiple POMs. At least one TS, e.g. the first TS, or the ARS can be adapted to measure data concerning a position and orientation of two or more POMs, e.g. a first POM fixedly attached of the MID and a second POM fixedly attached to the OHMD. In some examples, the first TS is adapted to measure data concerning a first POM and the second TS is adapted to measure data concerning a second POM.

In some embodiments, the POM is a magnetic marker, i.e. a marker that can be recognized, wherein the PO_{POM} preferably can be measured, using magnetic fields. The magnetic marker can comprise a coil, preferably three orthogonal coils.

In some embodiments, the POM is a visual and/or infrared marker, i.e. a marker that can be recognized, wherein the PO_{POM} preferably can be measured, using visible and/or infrared electromagnetic radiation. Preferably, such a POM can be adapted to reflect visible and/or infrared electromagnetic radiation.

In some embodiments, the visual and/or infrared POM comprises a vierbein, i.e. four spheres whose positions are fixed relative to one another. Preferably, the four spheres are reflective to visual and/or infrared electromagnetic radiation. Because the relative positioning of the four spheres is known, it is possible to determine the position and orientation of the vierbein based on data measured from almost any angle.

In some embodiments, the visual and/or infrared POM comprises an image pattern, preferably an image pattern comprising a plurality of vertices and edges such as e.g. a QR-code. The image pattern can e.g. be two dimensional or three dimensional. An image pattern can allow for determining a position and orientation of itself, which in turn can allow for determining a position and orientation of an object to which the image pattern is fixedly attached.

In some embodiments, two or more POMs, e.g. image patterns, are fixedly attached to the same object. Preferably, two or more POMs are attached to said object in such manner that they face in two or more different directions of the object. In an example, four or more POMs are attached to an object, preferably in cases where the TS comprises sensors facing to two or more directions. In some embodiments, six or more POMs are attached to an object, e.g. one or more to each side of a cuboid object. In an example, the use of multiple two-dimensional image patterns can allow for recognizing the position and orientation of a three-dimensional object.

In some embodiments, the ARS comprises a first TS, a second TS and a POM, wherein first TS as well as the second TS are adapted to measure data concerning the position and orientation of said POM. Thereby the PO_{POM} can be measured using two different TS, e.g. for use in a redundant system and/or for calibration of the ARS.

In some embodiments, a POM is fixedly attached to the MID and the TS is adapted to measure the PO_{POM}. Because of said fixation, tracking the PO_{POM} can allow the processing unit to calculate, possibly as well using calibration data, the PO_{MID}. In other words, the TS can measure data concerning the PO_{MID} by measuring the position and orientation of a POM fixedly attached to the MID. Preferably, the processing unit is adapted to transform the image taken by the MID from CS_{image} to CS_{MED} as well as to transform the so transformed image from CS_{MED} to CS_{OHMD} using the thereby calculated PO_{MID}.

In some embodiments, a first POM is fixedly attached to the MID, a second POM is fixedly attached to the OHMD and the TS is adapted to measure a position and orientation of the first POM ("PO_{1.POM}") and a position and orientation of the second POM ("PO_{2.POM}"). The processing unit is adapted to calculate, possibly as well using calibration data, the PO_{MID} from the PO_{1.POM}, and is further adapted to calculate, possibly as well using calibration data, the PO_{OHMD} from the PO_{2.POM}. Preferably, the processing unit is adapted to transform the image taken by the MID from CS_{image} to CS_{MED} using the calculated PO_{MID} and to transform the so transformed image from CS_{MED} to CS_{OHMD} using the calculated PO_{MID} and PO_{OHMD}.

Using POMs allows to easily modify existing MIDs so that they can be used in the ARS as proposed, namely by fixedly attaching one or more POMs to such a MID, especially since MIDs often are expensive and have a long lifespan.

In some embodiments, the TS is adapted to measure data concerning a position and orientation of object marker-less, i.e. without recognizing a specialized marker. For example, the TS can be configured for using shape recognition to recognize the position and orientation of an object, e.g. by using a scan and/or a CAD model of the real-world object as a virtual reference object. In an example, the MID is not rotationally symmetric and the TS is adapted to track the MID using shape recognition.

In some embodiments, the ARS comprises a camera, preferably a camera that is fixed relative to the OHMD. Such camera can allow the ARS to record the view of the bearer, e.g. for documentation. The ARS can be adapted to record the not-augmented view of the bearer and/or the augmented view of the bearer.

In some embodiments, the ARS is configured to save a still image of a displayed transformed image. Preferably, the ARS is configured to save a still image of the current view of the bearer, i.e. a still image of the real world (e.g. taken by a camera of the OHMD) onto which the currently displayed transformed image is overlaid. Such still images can e.g. be used for documentation. The ARS can comprise a saving means that is adapted to instruct the ARS to save a still image of a currently displayed transformed image.

In some embodiments, the ARS comprises an interruption means that is adapted to instruct the ARS to not display a transformed image on the OHMD. Said interruption means can e.g. be used when the bearer intends to see a non-augmented view.

In some embodiments, the ARS comprises a virtual button that is displayed on the OHMD and which e.g. can be overlaid onto the MID. For example, an intended action can be triggered, e.g. an image is saved, if the operator gazes at the virtual button for a prescribed amount of time. The ARS can be configured to display a circular progress indicator providing feedback regarding the imminence of the trigger to the operator during the gazing. Preferably, the ARS, e.g. the OHMD, comprises means for measuring data concerning the position of an eye, preferably of both eyes, of the operator, which can allow determining if the operator is gazing at a certain position, e.g. at a position where the virtual button is displayed. In another example, the ARS is configured to determine a gazing using the PO_{OHMD}. The ARS can be configured so that at least one function of the ARS can be triggered, preferably controlled, using the virtual button.

In some embodiments, the ARS comprises means for voice control, i.e. it is configured to receive and recognize voice commands. The ARS can be configured so that at least one function of the ARS can be triggered, preferably controlled, using voice commands.

In some embodiments, the ARS is configured so that at least one function of the ARS can be triggered, preferably controlled, using non-manual interaction, e.g. by using voice control and/or a virtual button. Controlling at least parts of the functions of the ARS using non-manual interactions can allow performing interventions using less personnel, thereby possibly saving costs and space in the operating room.

In some embodiments, the ARS, e.g. the OHMD, comprises a physical button that is configured trigger, preferably control, at least one function of the ARS. Preferably, the button is arranged at a location that is easily accessible to the bearer, e.g. on a side of the OHMD or a small hand-held device, e.g. a remote control.

In some embodiments, the ARS comprises a medical or surgical tool, such as a syringe, needle or a pointer, and the ARS is configured to display the surgical tool in a highlighted manner on the OHMD. Preferably, the ARS is configured to highlight the surgical tool in the displayed image.

In some embodiments, the medical and/or surgical tool comprises tool-recognition means that allows the ARS, preferably the TS and/or the processing unit (e.g. using the data comprised in the medical image), to recognize said tool, which e.g. can allow the ARS to highlight the said tool in the displayed image. In an example, the surgical tool comprises a material that is easily recognizable on the respective medical image, e.g. a metal. The tool-recognition means can comprise a POM.

Furthermore, methods that are represented by the embodiments of ARS, or parts thereof, disclosed herein are proposed.

Furthermore, a method for creating an augmented reality by displaying an image taken by the MID, preferably in quasi real-time, on the OHMD comprising the steps of:
- taking an image using the MID;
- measuring data concerning a position and orientation of at least one object of the ARS;
- transforming the image taken by the MID from the CS_{image} to the CS_{OHMD} using the measured data; and
- displaying the transformed image in a position, orientation and scale that corresponds to the perspective of a PO_{OHMD} in quasi-real-time on the OHMD.

The method can be performed in the order as written or in any other technically appropriate order. For example, the step of taking the image can be performed before, while, and/or after the step of measuring data concerning the at least one object is performed. The method can be iterated to - at least quasi-continuously - adjust the displayed image to the current PO_{OHMD}, preferably wherein the step of taking an image is not iterated in case the image to be displayed is a still image.

The said at least one object can comprise the OHMD. By tracking the OHMD (i.e. measuring the PO_{OHMD} over time), the image (or images) can continuously be transformed to fit to the current perspective of the OHMD.

The said at least one object can comprise the MID. By tracking the MID (i.e. measuring the PO_{MID} over time), images continuously taken by the MID can be transformed and displayed.

In some variants, the step of measuring data comprises measuring data concerning the PO_{OHMD} relative to the PO_{MID}. Preferably, the variant comprises calculating the PO_{OHMD} relative to the PO_{MID}, for example by calculating the PO_{OHMD} and the PO_{MID} relative to a chosen reference system, e.g. a world reference. The reference system can be chosen according to a PO_{OHMD} at a chosen time, e.g. at the time the OHMD is first started during a session, i.e. when the OHMD is initialized.

In some variants, the step of measuring data is performed by one or more TSs. At least one of the one or more TSs can be fixed relative to the OHMD.

In some variants, the method comprises measuring data concerning a spatial acceleration and a rotation rate of at least one object, preferably the OHMD and/or the MID. Preferably, the step of transforming the image taken by the MID from the CS_{image} to the CS_{OHMD} is performed using an estimate of the position and orientation of the at least one object, whereby said estimate is calculated using:
- the measured data concerning a spatial acceleration and a rotation rate of the at least one object; and
- an earlier determined position and orientation of the at least one object.

The variation of the position and of the orientation of the at least one object over time can be calculated from its spatial acceleration and rotation rate. If a position and orientation of said at least one object at a first time and the variation of the position and of the orientation of said at least one object between the first time and a later second time is known, it is possible to calculate the position and orientation of said at least one object at the second time. Measurements of spatial acceleration and a rotation rate can be subject to significant tolerances, and thus the results of such method of calculation can be perceived as estimates. A Kalman filter that takes into account an earlier state of the system and the measured variation can be employed to increase the precision of such estimates.

Spatial acceleration and rotation rate can be measured using an IMU that is comprised in the object to be tracked. Such IMU is considered a highly reliable system in the sense that its measurements are only rarely interrupted, which encourages its use in a back-up system.

In some variants, the method comprises switching from
- a first way of performing the step of transforming the image taken by the MID from the CS_{image} to the CS_{OHMD}
   to
- a second way of performing the step of transforming the image taken by the MID (2) from the CS_{image} to the CS_{OHMD}
upon the occurrence of a triggering event. An example of such a triggering event can be that a measurement that is used in the first way is deemed unreliable, e.g. with respect to accuracy and/or latency. Choosing the second way of transforming the image can allow for continued usage of the method in case where the measurements on which the first way is based are corrupted. Preferably, the first way is a way of high precision, while the second way is a way of high reliability with respect to the availability of the measured data.

In some variants, the second way comprises estimating the position and orientation of the at least one object using
- the measured data concerning a spatial acceleration and a rotation rate of the at least one object; and
- an earlier determined position and orientation of the at least one object.

Preferably, the first way does not comprise an estimation of this kind.

Furthermore, embodiments of the ARS, or parts thereof, adapted to perform the methods disclosed herein are proposed.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a state-of-the-art system for in-situ displaying medical images;
- Fig. 2: shows a first embodiment of an ARS;
- Fig. 3: shows the ARS of figure 2 with respective coordinate systems;
- Fig. 4: shows an ARS comprising an X-ray;
- Fig. 5: shows a second embodiment of an ARS;
- Fig. 6: shows an ARS using shape recognition;
- Fig. 7: shows an AR image comprising an ultrasound image;
- Fig. 8: shows an AR image comprising an X-ray image;
- Fig. 9: shows an ARS with a first TS and a second TS;
- Fig. 10: shows a system for calibration concerning the POM of a MID;
- Fig. 11: shows two possible positions of a user with respect to a TS;
- Fig. 12: shows how to calculate the PO_{MID} using an IMU;
- Fig. 13: shows an OHMD;
- Fig. 14a-c: show a gaze button;
- Fig. 15: shows a processing unit;
- Fig. 16: shows a MID comprising an IMU;
- Fig. 17: shows a method for displaying an augmented reality using a sequence of images;
- Fig. 18: shows a method for displaying an augmented reality using a single image;
- Fig. 19: shows a method of transforming the image;
- Fig. 20a-b: show a method using two different ways of transformation;
- Fig. 21: shows a method of calculating an estimate of the position and orientation of an object.

### DESCRIPTION OF PREFERRED EMBODIMENTS

In the following, the invention is primarily exemplified with a view towards ultrasound and X-ray imaging. However, of course the invention can be used with other medical imaging technologies.

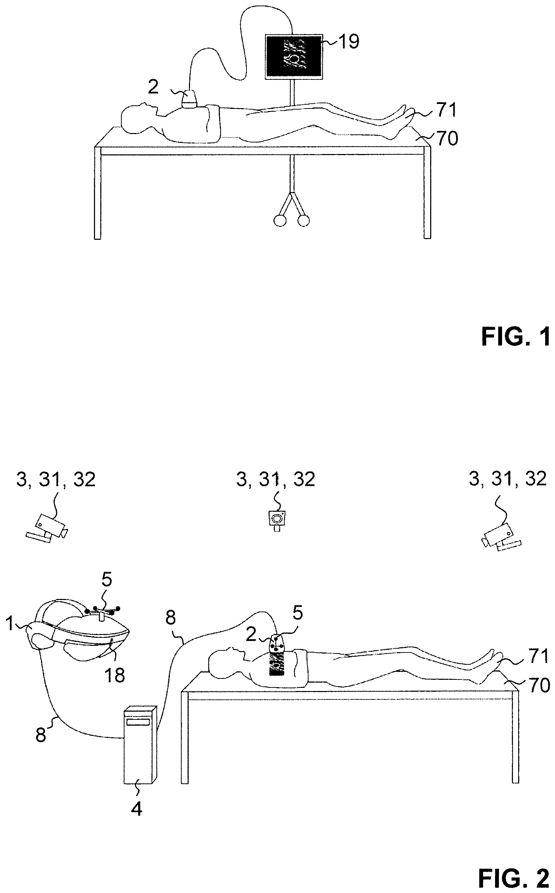

**Figure 1** shows a state-of-the-art system for in-situ displaying ultrasound images during medical interventions or diagnostic procedures. The ultrasound device 2 comprises an ultrasound probe 20 for taking the image and is connected to a monitor 19 that is arranged at a side of the operating table 70 on which the patient 71 lays. An ultrasound image is displayed on the monitor 19 in a position and orientation in which the image was taken ("PO_{image}"). In order to perform a diagnosis or an intervention using the ultrasound images, the operator must look at the monitor 19 and thus away from the spot at which they perform the intervention. This procedure is counter-intuitive and thus requires a lot of skill and experience to perform with the high level of accuracy and safety that are desired for medical purposes.

To improve the applicability, safety and/or reliability of medical imaging especially during interventions, an augmented reality system ("ARS") is proposed that comprises
- an optical head mounted display 1 ("OHMD") that is designed to be worn on the head and to display images in the field of view of its bearer,
- a medical imaging device 2 ("MID") that is designed to take a medical image, preferably record a series of images,
- a tracking system ("TS") 3 that is adapted to measure data concerning the position and orientation of at least one object (e.g. the MID 2 and/or the OHMD 1) and
- a processing unit 4 that is configured to transform the image taken by the MID 2.

The ARS is configured to display the transformed image in the field of view of the bearer of the OHMD 1, thereby creating an augmented reality ("AR") image. The displayed image is a transformation of the original image such that when displayed to the bearer of the OHMD 1, the displayed image appears to be at the position and orientation of the image ("PO_{image}") as seen from the perspective of the position and orientation of the OHMD 1 ("PO_{OHMD}"). In other words, the ARS is adapted to display the transformed image on the OHMD 1 in a position, orientation and scale that corresponds to the perspective of the current PO_{OHMD}. Preferably, the ARS is adapted to display a sequence of transformed images in chronological order such that the displayed image adapts to the currently taken image and the current perspective of the PO_{OHMD} in quasi-real-time.

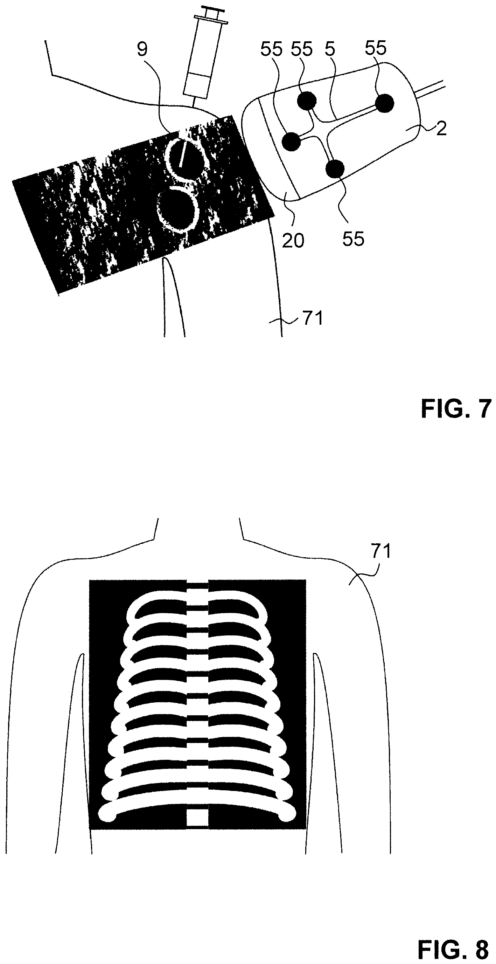

An example of the AR image as seen by the bearer of the OHMD is shown in **Figure 7****.** The bearer sees the reality in front of them, for example a patient 71, a MID 2 (here in form of an ultrasound device) and a surgical tool 9 (here in form of a syringe), with a part of the view of the real world being overlaid with an image displayed on the OHMD 1. The image is not displayed as it was originally taken, which typically is from the perspective of the sensor of the MID 2. Instead, a transformation of the image is displayed in a position, orientation and scale that corresponds to the perspective of the PO_{OHMD}. Thus, the bearer has the impression to see the real world overlaid with a holographic medical image that adjusts to the position of the bearer's view. This allows the bearer to use medical imaging in an intuitive manner, which can greatly support medical diagnostic procedures and surgical or non-surgical interventions.

In the example of figure 7, a needle of a syringe 9 is inserted into the patient's body. The bearer can see the syringe 9 as well as a first ('upper') part, namely the part that is not inserted into the body yet, and a third ('lower') part, namely the part that is inserted into the body and that is visible in the ultrasound image, of the needle. A second ('middle') part of the needle, namely the part that is inserted into the body but is not visible in the displayed section of the ultrasound image, is not visible to the bearer. The proposed ARS allows an operator to look at the location at which the syringe 9 is inserted while perceiving said location in form of a medical image.

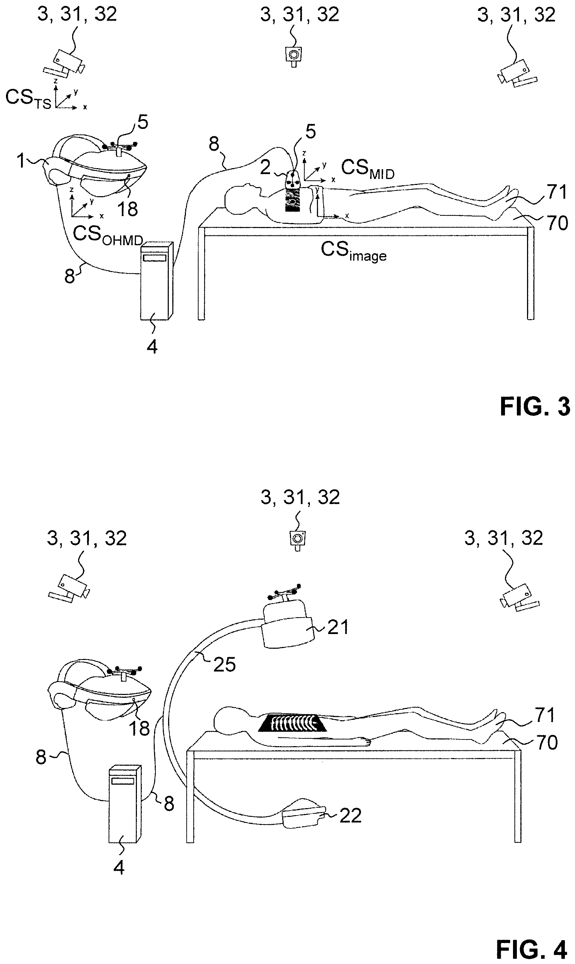

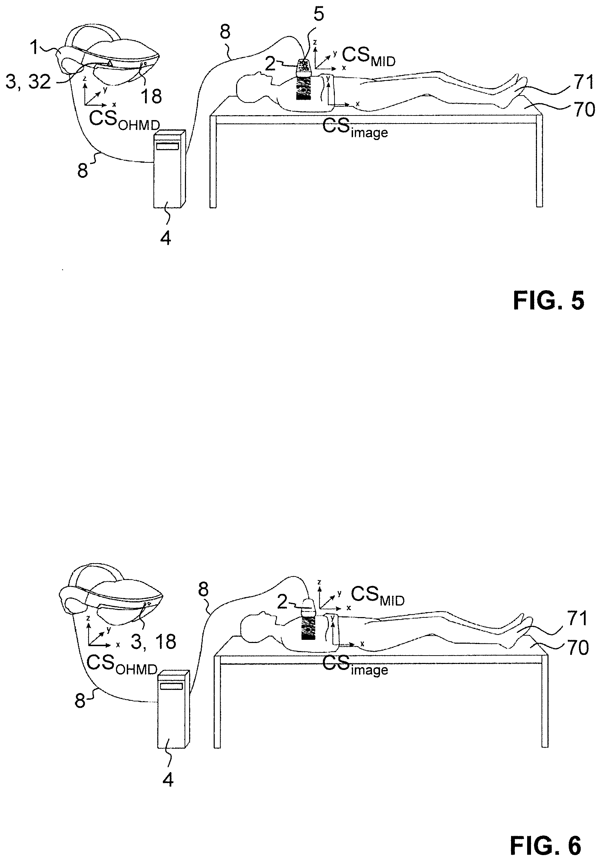

**Figure 8** shows another example of an AR image as visible to the bearer of the OHMD 1, but this time the overlaid image is an X-ray image. An X-ray image is a two-dimensional projection of the three-dimensional segment between an X-ray-emitter 21 and an X-ray-plate 22. Thus, an orientation and a scale can be allocated to the X-ray image, but not an unambiguous position. The ARS is adapted to make a choice concerning the position, preferably a choice that approximates the intuitive understanding of the image. The X-ray can e.g. be chosen to be displayed as to match the anatomy of the respective body part, e.g. halfway through the body as indicated in **figure 4****.** The ARS can comprise adjusting means, e.g. operated using hardware means 15 and/or software means, for adjusting the choices made by the ARS, e.g. to display the X-ray image of figure 5 at a higher or lower position.

The transformation comprises transforming the image from a first coordinate system to a second coordinate system using data measured by the TS 3, for example from a coordinate system of the image ("CS_{image}") to the coordinate system of the OHMD 1 ("CS_{OHMD}"). Preferably, the transformation comprises at least transforming the image from the CS_{image} to a coordinate system of the MID 2 ("CS_{MID}") and from the CS_{MID} to the CS_{OHMD}.

**Figure 2** shows a first embodiment of the proposed ARS, wherein the TS 3 is not fixed relative to the PO_{OHMD}. Instead, the TS 3 can be static, e.g. by being fixedly installed in the operating room. The hardware components of the ARS (e.g. the OHMD 1, the MID 2, the TS 3 and/or the processing unit 4) communicate with each other via wireless and/or via wired connections 8. The TS 3 is adapted to measure data concerning a position and orientation of the MID ("PO_{MID}") as well as the PO_{OHMD}.

The MID 2 and the OHMD 1 are each fixedly attached to a position and orientation marker 5 ("POM"), which in this example are both realized as infrared-reflective vierbeins, i.e. a device comprising four spheres 55 at a predefined position from each other as can be seen in figure 7. The TS 3 comprises emitters 31 for infrared light, i.e. electromagnetic radiation with wavelengths between 700 nanometers to 1 millimeter, and detectors 32 in form of cameras. Preferably, near infrared light, i.e. electromagnetic radiation with wavelengths between 700 nanometers to 1.4 micrometer, is used. The TS 3 measures the infrared light reflected by the POMs 5, which can allow the ARS to recognize the spheres 55 and thus to determine the position and orientation of a POM 5 ("PO_{POM}") and thereby, e.g. by using calibration data, the position and orientation of an object to which said POM 5 is fixedly attached to. In the example of figure 2, the ARS is adapted to determine the PO_{MID} and the PO_{OHMD} using data concerning the PO_{MID} resp. the PO_{OHMD} measured by the TS 3.

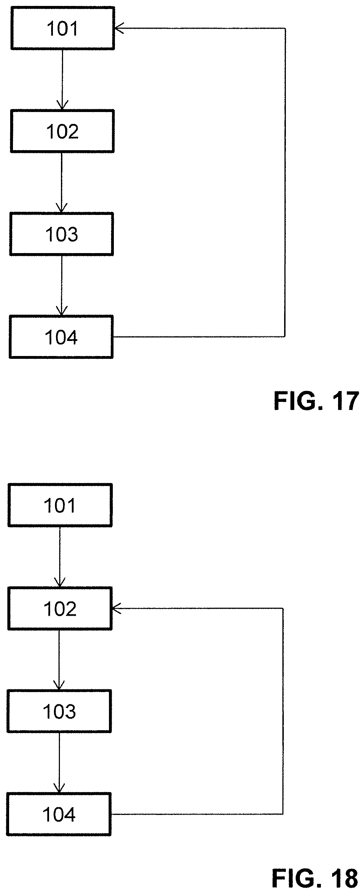

In some embodiments, the ARS is adapted to perform the method for creating an augmented reality according to the methods shown in **figure 17** **and/or** **figure 18****,** which comprise:

| | |
|---|---|
| Step 101: | taking an image (i.e. the medical image) using the MID 2; |
| Step 102: | measuring data concerning a position and orientation of at least one object of the ARS, e.g. the MID 2 and/or the OHMD 1; |
| Step 103: | transforming the image from CS_{image} to CS_{OHMD} using the measured data in Step 102; and |
| Step 104: | displaying the transformed image in a position, orientation and scale that corresponds to the perspective of the "PO_{OHMD}", preferably in quasi-real-time, on the OHMD 1. |

Of course, step 101 and step 102 can be performed simultaneously or in any order. Preferably, the data measured in step 102 allow for a determination of the PO_{image} (resp. the PO_{MID}) relative to the PO_{OHMD}. The measurements of step 102 can be made by one or multiple TSs 3, 3'. Step 103 can comprise using a Kalman filter.

The ARS can further be adapted to iterate this process. For example, if a still image shall be displayed, the steps 102 - 104 are iterated as shown in figure 18 so that the ARS can adapt the transformation of the still image according to the current perspective of the OHMD 1. This method can e.g. be used for X-ray images, which - due to the radiation exposure - are typically only taken once or a limited number of times. The ARS can be adapted to store multiple still images and the respective position and orientation of the respective MIDs 2, between which the operator can switch. Upon each switch, the steps 102 - 104 are repeated so as to adjust the newly chosen image to the current perspective of the operator; and thereafter the steps 102 - 104 are again iterated to adjust the chosen image to the changing perspective of the operator.

In another example, if a sequence, preferably an image stream/video, of images shall be displayed, the steps 101 - 104 are iterated as shown in figure 18 so that the ARS can adapt the transformation of the currently taken image according to the current perspective of the OHMD 1. This method can e.g. be used for continuously taken ultrasonic images during an intervention. Taking the image and measuring data concerning of at least one object of the ARS can be synchronized or be performed at different frequencies. In the latter case, the last taken image is adjusted to the current perspective of the OHMD 1.

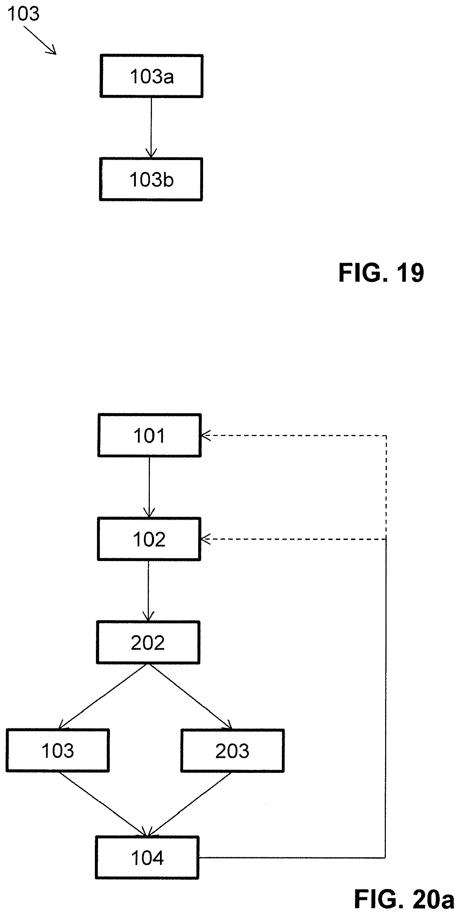

As indicated by the coordinate systems in **figure 3****,** the ARS according to the first embodiment can be adapted to transform the image according to step 103 as shown in **figure 19****,** which comprises:

| | |
|---|---|
| Step 103a: | transforming the image from CS_{image} to CS_{MID}; |
| Step 103b: | transforming the image from CS_{MID} to CS_{OHMD}. |

This can allow transforming the image from the perspective in which the image was originally taken (i.e. the perspective according to the PO_{image}) to the perspective of the OHMD 1 (i.e. the perspective according to the PO_{OHMD}). Preferably, each of the steps is expressed as a matrix, e.g. a 4x4 matrix, and the composition of the steps is expressed as a multiplication of the respective matrices.

In the example shown in figure 3, the TS 3 measures data concerning the PO_{MID} and the PO_{OHMD} relative the TS 3 in step 102, which in step 103b are used to transform the image from CS_{MID} to CS_{OHMD}. Since in this example the TS 3 effectively measures the position and orientation of the respective POMs 5, step 103a can comprise using calibration data concerning the position and orientation of the POM 5 that is fixedly attached to the MID 2; and step 103b can comprise using calibration data concerning the position and orientation of the POM 5 that is fixedly attached to the OHMD 1. In practice, steps 103b can comprise transforming CS_{MID} to a coordinate system of the TS 3 ("CS_{TS}") and transforming the image from CS_{TS} to CS_{OHMD}, wherein CS_{TS} is a reference coordinate system of the TS 3. In the embodiment of figure 3, the TS 3 is fixed relative to the world (e.g. static in an operating room) and the CS_{TS} can be chosen as a world reference system.

While figure 3 shows an example wherein the MID 2 is an ultrasound device, figure 4 shows an example of the first embodiment wherein the MID 2 is an X-ray imaging device that comprises a C-arm 25, which comprises the X-ray emitter 21 at one end and the X-ray plate 22 at the other end.

**Figure 5** shows a second embodiment of the proposed ARS, wherein the TS 3 is fixed relative to the PO_{OHMD}. A POM 5, in this example an image pattern, is fixedly attached to the MID 2. The TS 3 comprises a detector 32 in form of a camera and the ARS is adapted to calculate the position and orientation of the image pattern 5 using the data measured by the camera 32.

In the example of figure 5, the ARS is adapted to measure data concerning the PO_{MID} relative to the TS in step 102, i.e. relative to the PO_{OHMD}. In case where a still image shall be displayed, step 102 can amount to measuring the position and orientation of the OHMD relative to the one position and orientation in which the still image was taken.

**Figure 6** shows an ARS that differs from that of figure 5 in that the ARS does not comprise POMs 5. Instead, the ARS is adapted to track the MID using shape recognition, e.g. it is adapted to recognize the MID 2 itself or parts thereof. In a similar fashion, the first embodiment as e.g. shown in figure 3 can as well be realized without POMs 5 and the ARS can be adapted to track the MID and the OHMD using shape recognition. In this case, the TS 3 comprises the camera 18 integrated into the OHMD 1 that is used as a recognition means for the shape recognition.

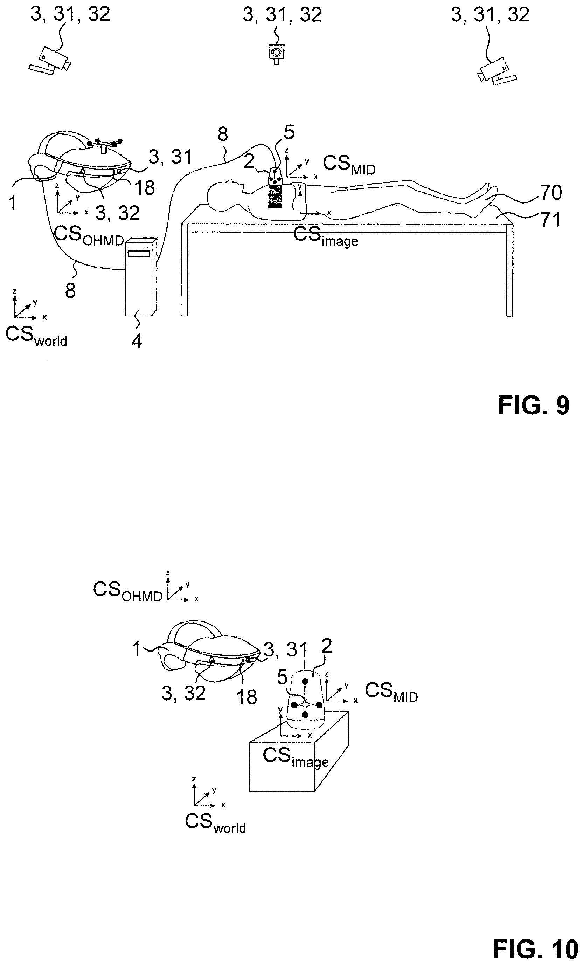

**Figure 9** shows an ARS that comprises two TSs 3, 3', wherein a first TS 3 is not fixed relative to the PO_{OHMD} and a second TS 3' is fixed relative to the PO_{OHMD} in that it is integrated into the OHMD 1. The first TS 3 operates essentially in the same way as the TS shown in figure 3, and the second TS 3' operates essentially in the same way as the TS shown in figure 5, but here the second TS 3' uses infrared based measurements to track the infrared-reflective vierbein 5 that is fixedly attached to the MID 3.

An ARS comprising two or more TSs 3, 3' can be adapted to calculate the position and orientation of one or more objects with increased precision. For example, the processor unit 5 can be adapted to calculate the PO_{MID} using data measured by the first TS 3 as well as data measured by the second TS 3', thereby fusing the data measured by the two TSs 3, 3'. Preferably, the processor unit 5 can be adapted to calculate, e.g. as part of step 103, the PO_{MID} using a Kalman filter that fuses data measured by the first TS 3 and data measured by the second TS 3'.

An ARS comprising two or more TSs 3, 3' can be adapted to calculate the transformed image with increased precision. For example, the processor unit 5 can be adapted to calculate the transformed image using data measured by the first TS 3 as well as to calculate the transformed image using data measured by the second TS 3' (e.g. according to the method shown in figure 19), thereby fusing the data measured by the two TSs 3, 3'. For example, the processor unit 5 can be adapted to calculate, e.g. as part of step 103, the transformed image using a weighted average of the transformed image as calculated using data measured by the first TS 3 and of the transformed image as calculated using data measured by the second TS 3'.

An ARS comprising two or more TSs 3, 3' can be adapted to conduct plausibility checks. For example, the ARS can be adapted to transform the image using data measured by the first TS 3 and is further adapted to use, e.g. as part of step 102 or step 103, data measured by the second TS 3' to check if the data measured by the first TS 3 are plausible. Of course, the role of the first TS 3 and the second TS 3' in this context is interchangeable.

An ARS comprising two or more TSs 3, 3' can be adapted to determine calibration data. For example, the ARS can be adapted to determine calibration data with respect to the exact attachment of POM 5 to the MID 2 or the OHMD 1 and determine the PO_{MID} resp. the PO_{OHMD} using data concerning the position and orientation of the respective POM 5 and the respective calibration data.

As indicated in figure 9, calibration data can be determined using the environment ("world") as a reference coordinate system ("CS_{world}"). Two or more TSs 3, 3' can measure data concerning the same environment, thereby determining their respective position and orientation relative to CS_{world}. This calibration technique is preferably used when the position and orientation and orientation of at least one of the TSs 3, 3' relative to the PO_{MID} or PO_{OHMD} is known. In the example shown in figure 9, a simultaneous measurement of the environment using the first TS 3 and the second TS 3' allows the ARS to determine the position and orientation of the two TS 3, 3' relative to each other; and a measurement of the first TS 3 allows the ARS to determine the position and orientation of the POM 5 that is fixedly attached to the OHMD 1. Thereby, calibration data concerning the PO_{POM} relative to the PO_{OHMD} can be determined, if the position and orientation of the OHMD 1 relative to the second TS 3' are known. Using this calibration data, the ARS can calculate the PO_{OHMD} using data concerning the PO_{POM} as measured by the first TS 3 and transform the image using said calculated PO_{OHMD}. In this way, the second TS 3', which was used during calibration, does not necessarily have to be used during regular operation of the ARS (i.e. after calibration has been completed).

An arrangement for calibrating the PO_{image} resp. the PO_{MID} relative to a POM 5 that is fixedly attached to the MID 2 is shown in **figure 10****.**

An ARS comprising two or more TSs 3, 3' can be adapted to calculate the transformation of the image using the data measured by a first TS 3 in first mode and to calculate the transformation of the image using the data measured by a second TS 3' in a second mode. For example, the ARS can be adapted to usually calculate the transformation of the image in a way using data measured by the first TS 3. However, the ARS can be adapted to instead calculate the transformation in a way using data measured by the second TS 3'. The ARS can be adapted to switch to this second mode during periods where the first TS 3 does not provide sufficiently reliable data, e.g. where the PO_{MID} or PO_{OHMD} cannot be determined using the data measured by the first TS 3. The ARS can e.g. be adapted to calculate the transformation using measurements of the first TS 3 and using measurements of the second TS 3' in the first mode, e.g. for increasing the precision of the calculation of the transformation and/or of a position and orientation of an object, and to calculate the transformation either using measurements of the first TS 3 or using measurements of the second TS 3' in the second mode if the measurements of the other is deemed unreliable.

The ARS can be adapted to perform the method as shown in figure 20a, namely:

| | | |
|---|---|---|
| Step 101: | taking an image (i.e. the medical image) using the MID 2; | |
| Step 102: | measuring data concerning a position and orientation of at least one object of the ARS, e.g. the MID 2 and/or the OHMD 1, preferably using two different TSs 3, 3'; | |
| Step 202: | decide if a triggering event has occurred; | |
| If NO: perform step 103: | | transforming in a first way the image from CS_{image} to CS_{OHMD} using the measured data in Step 102; |
| If YES: perform step 203: | | transforming in a second way the image from CS_{image} to CS_{OHMD} using the measured data in Step 102; and |
| Step 104: | displaying the image as transformed in step 103 or in step 203 in a position, orientation and scale that corresponds to the perspective of the "PO_{OHMD}", preferably in quasi-real-time, on the OHMD 1. | |

Again, step 101 and step 102 can of course be performed simultaneously or in any order.

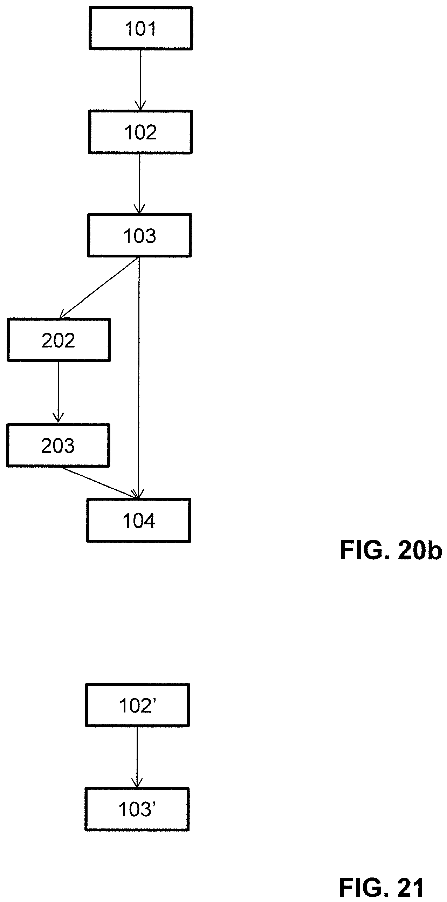

The method can further comprise iterating the process, thereby possibly restarting at step 101 to display a sequence of images, or at step 102 to display a still image. The triggering event can occur in case step 103 malfunctions, such as displayed in figure 20b.

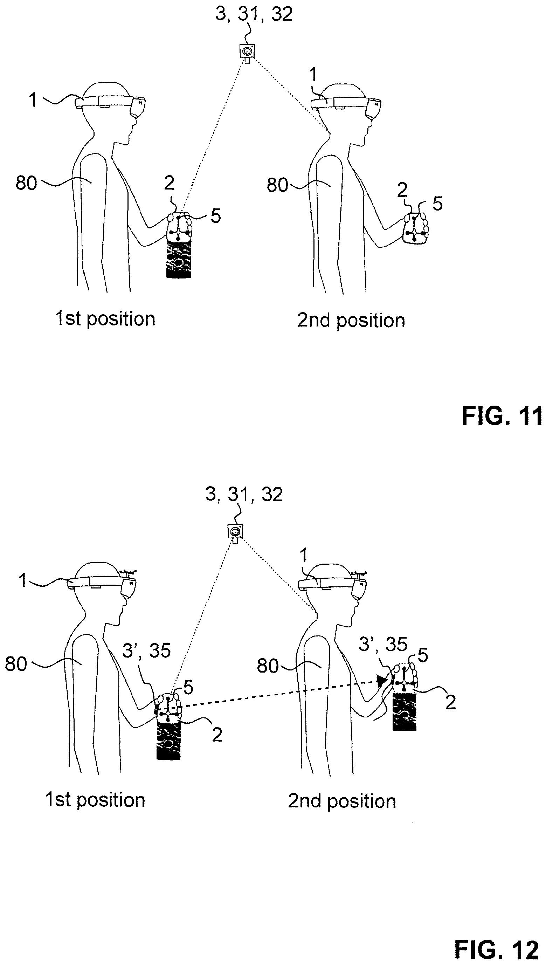

For example, in **figure 11**, the TS 3 is adapted for tracking the MID 2 based on infrared-reflection of the vierbein 5 that is fixedly attached thereto; a system which is typically considered to be particularly precise. In the first position of the operator, the path from the vierbein 5 to the detector 32 of the TS 3 is unobstructed, such that the TS 3 can track the vierbein 5 and thus the MID 2 and the transformation of the image can be calculated based of this tracking in a first way. In the second position however, the body of the operator 80 blocks the path from the vierbein 5 to the detector 32 of the TS 3, which can lead to a disruption of the tracking of the TS 3 and thus to the ARS not being able to calculate the transformation of the image using the first way. This can be classified as a triggering event. The ARS can be adapted to, in this case, calculate the transformation of the image in a second way, preferably using data measured by a second TS 3'. The second TS 3' is preferably chosen to be a particularly reliable system, e.g. comprising sensor means whose measurements cannot easily be blocked.

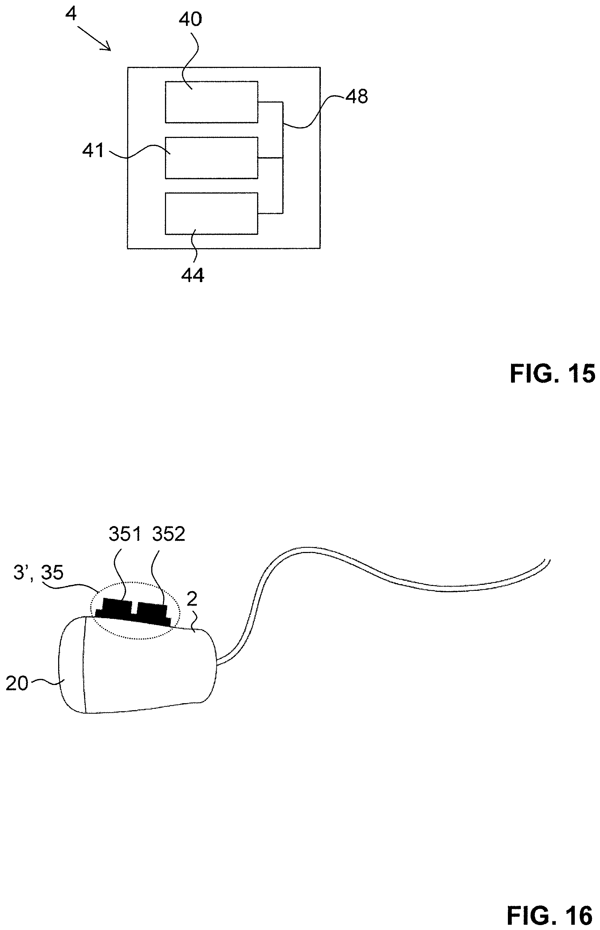

**Figure 16** shows a MID 2 comprising an inertial measurement unit 35 ("IMU") that is adapted to measure data concerning a spatial acceleration and a rotation rate of the MID 2. The IMU 35 comprises an accelerometer 351 and a gyroscope 352 The IMU 35 can e.g. be part of a TS 3, preferably of a second TS 3'.

The data measured by the IMU 35 can allow for calculating the relative movement and rotation of the MID 2. In other words, data measured by the IMU 35 itself do not necessarily allow for the determination of the absolute PO_{MID}, but rather the relative movement of the MID 2 by a certain length along a certain direction, and the relative rotation of the MID 2 by a certain angle along a certain axis.

**Figure 12** shows an example of how to calculate an estimation of the PO_{MID} using a second TS 3' comprising an IMU 35. The MID 2 is normally tracked using the camera 32 of the first TS 3. When the view of the camera 32 towards the MID 2 is obstructed (e.g. by the operator, or any object with material properties that are opaque with respect to the radiation used by the TS), the TS 3 is no longer capable of tracking the MID 2. However, an IMU 35 of the second TS 3' that is fixedly attached to the MID 2 continues to measure the acceleration and a rotation rate of the MID 2, from which the ARS is able to calculate the variation of the PO_{MID}. By taking into the account the PO_{MID} that was last determined using data measured by the first TS 3, and by continuously updating the PO_{MID} using data measured by the IMU 35, the ARS is able to calculate a prediction of the current PO_{MID}. In many cases, most notably when the ARS has to rely solely on the data measured by the IMU 35 for extended periods of time, the precision of this calculation can be limited, and the result should therefore be perceived as an estimate of the PO_{MID}.

The ARS can be adapted to transform the image according to the method as shown in **figure 21****,** which comprises:

| | | |
|---|---|---|
| Step 102' | measuring data concerning a spatial acceleration and a rotation rate of at least one object, preferably at least of the MID 2, and | |
| Step 103' | transforming the image taken by the MID 2 from the CS_{image} to the CS_{OHMD} using an estimate of the position and orientation of the at least one object, preferably at least of the MID 2, that is calculated using | |
| | | ▪ the measured data concerning a spatial acceleration and a rotation rate of the at least one object; and |
| | | ▪ an earlier determined position and orientation of the at least one object. |

Step 102' and step 103' can be comprised in or replace step 102, step 103 and step 203, respectively, in the methods of figures 17, 18, 20a or 20b. Preferably, step 103' replaces step 203 in the methods of figure 20a resp. figure 20b. Step 103' can comprise using a Kalman filter.

The methods of figure 20a and/or figure 20b can further comprise storing a position and orientation of the at least one object prior to the triggering event, e.g. as determined while performing the first way, to be used as the earlier determined position and orientation of the at least one object in step 103'.

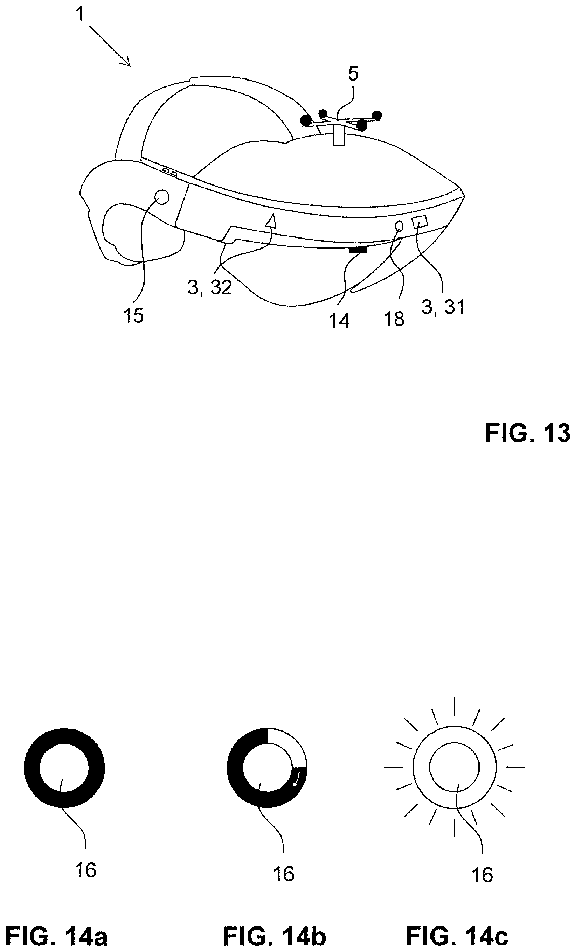

**Figure 13** shows an OHMD 1 that can e.g. use curved mirrors or waveguides to display an artificial image in the field of view of its bearer. The OHMD 1 can comprise hardware means 15 for controlling the ARS, e.g. a button.

A TS 3 comprising an infrared emitter 31 and an infrared detector 32 is integrated in the shown OHMD 1. Preferably, the TS 3 comprises two or more infrared detectors 32, which can improve the area and/or the precision of the tracking. In addition to the TS 3, the shown OHMD 1 comprises a camera 18, which can be used as a second TS 3' (e.g. as a monitoring means for monitoring the reliability of the data measured by the first TS 3) and/or to record the perspective of the bearer (e.g. for documentation).

Pupil sensors 14 allow tracking the position of the pupils of the eyes of the bearer, which can be used to adjust the displayed image to the bearer's physiognomy. This can e.g. entail the adjustment of the stereo-displaying in accordance with the bearer's interpupillary distance, and/or the scaling of the image in accordance with the distance of the bearer's pupils from the mirrors.

The pupil sensors 14 can also allow for implementing a gaze control, e.g. in the form of a gaze button. An example of a possible design of a gaze button 16 as displayed to the bearer of the OHMD 1 is shown in **figures 14a to 14c****.** Here, the gaze button 16 is implemented as a circular progress bar, which indicates the duration of the bearer's gaze. It can be configured in such a way that the bearer is required to gaze at a certain position, preferably a position where the gaze button is displayed, for a prescribed amount of time, e.g. for 2 seconds, until an action is triggered. Figure 14a shows the gaze button 16 in a default state in which the gaze button 16 is displayed in a neutral background color. Once the bearer of the OHMD starts gazing at the gaze button 16, it starts to fill up with a contrasting foreground color, for example in a counter-clockwise direction as shown in figure 14b. If the bearer looks away from said certain position, the gaze button 16 quickly resets to its default state. However, if the gaze is held for the prescribed amount of time without interruption, the foreground color will fill the entire button and an additional visual cue is provided to indicate that a certain action has been triggered, e.g. by a flashing light, as indicated in figure 14c. Of course, sensor means other than the pupil sensor 14 can be used for triggering the gaze button 16. For example, the ARS can be configured to determine a gazing using the PO_{OHMD}, wherein the ARS preferably assumes that the gaze of the bearer of the OHMD 1 is in the centre of the field of view of the OHMD 1.

**Figure 15** shows a processing unit 4 comprising a processor (CPU) 40 and a volatile (e.g. RAM) memory 41 and/or a non-volatile (e.g. a hard disk) memory 44, wherein the processor 40 communicates with the memory modules 41, 44 using one or more data buses 48.

**LIST OF REFERENCE SIGNS**

| | | | |
|---|---|---|---|
| 1 | optical head mounted display ("OHMD") | 21 | X-ray emitter |
| | | 22 | X-ray plate |
| 2 | medical imaging device ("MID") | 25 | C-arm |
| | | 31 | emitter |
| 3 | tracking system ("TS") | 32 | detector |
| 3' | second TS | 35 | inertial measurement unit ("IMU") |
| 4 | processing unit | | |
| 5 | position and orientation marker ("POM") | 351 | accelerometer |
| | | 352 | gyroscope |
| 7 | hospital room | 40 | CPU |
| 8 | wired connection | 41 | volatile memory |
| 9 | surgical tool | 44 | non-volatile memory |
| 14 | pupil sensor | 48 | bus |
| 15 | hardware button | 55 | sphere (part of a vierbein) |
| 16 | gaze button | 70 | operating table |
| 18 | camera | 71 | patient |
| 19 | monitor (prior art) | 80 | operator |
| 20 | ultrasound probe | | |

## Claims

1. An augmented reality system ("ARS") for displaying a medical image in quasi-real-time, comprising
- an optical head mounted display (1) ("OHMD") that is designed to display images;
- a medical imaging device (2) ("MID") that is designed to take an image;
- a first tracking system (3) ("TS") that is adapted to measure data concerning a position and orientation of at least one object of the ARS; and
- a processing unit (4) that is configured to transform an image taken by the MID (2) from a coordinate system of the image taken by the MID (2) ("CS_{image}") to a coordinate system of the OHMD (1) ("CS_{OHMD}") using data measured by the first TS (3),
wherein the ARS is adapted to display the transformed image in quasi-real-time on the OHMD (1) in a position, orientation and scale that corresponds to the perspective of a position and orientation of the OHMD (1) ("PO_{OHMD}").

2. The ARS of claim 1, wherein the ARS comprises a second TS (3') adapted to measure data concerning a position and orientation of at least one object, preferably at least of the MID (2) and/or the OHMD (1).

3. The ARS of claim 2, wherein the second TS (3') comprises an inertial measurement unit (35) ("IMU") that is fixedly attached to the MID (2) and that is adapted to measure data concerning a spatial acceleration and a rotation rate of the MID (2),
wherein the processing unit (4) is preferably configured
- to estimate the current position and orientation of the image taken by the MID (2) ("PO_{image}") using the data measured by the IMU (35) and an earlier determined position and orientation of the image taken by the MID (2) calculated using data measured by the first TS (3); and
- to transform the image taken by the MID (2) from the CS_{image} to the CS_{OHMD} using the estimated PO_{image}.

4. The ARS of one of the preceding claims, wherein the processing unit (4) is configured to transform the image taken by the MID (2) from the CS_{image} to the CS_{OHMD} according to a first way and according to a second way.

5. The ARS of claim 4 and one of claims 2 or 3,
- wherein the first way comprises transforming the image taken by the MID (2) from the CS_{image} to the CS_{OHMD} using data measured by the first TS (3), and
- wherein the second way comprises transforming the image taken by the MID (2) from the CS_{image} to the CS_{OHMD} using data measured by the second TS (3').

6. The ARS of one of the claims 4 or 5, wherein the ARS is configured
- to normally operate in a first mode in which the transformed image that is displayed is calculated according to the first way, and
- to switch to a second mode in which the transformed image that is displayed is calculated according to the second way upon the occurrence of a triggering event,
and wherein the occurrence of the triggering event preferably comprises that measurements on which the first way is based are deemed unreliable.

7. The ARS of one of the preceding claims, wherein the ARS comprises a position and orientation marker (5) ("POM"), and wherein at least the first TS (3) is adapted to measure data concerning a position and orientation of the POM (5); and wherein the POM (5) is preferably attached fixedly to the MID (2) or the OHMD (1).

8. The ARS of one of the preceding claims, wherein the MID (2) comprises
- an ultrasound probe (20), and/or
- an X-ray emitter (21) and an X-ray plate (22).

9. The ARS of one of the preceding claims, wherein the PO_{OHMD} is fixed relative to the first TS (3).

10. The ARS of one of claims 1 to 8, wherein the PO_{OHMD} is not fixed relative to the first TS (3),
and wherein the first TS (3) preferably is adapted to measure data concerning the PO_{MID} and data concerning the PO_{OHMD}.

11. A method for creating an augmented reality by displaying an image taken by a medical image device ("MID") in quasi real-time on an optical head mounted display ("OHMD") comprising the steps of:
- taking an image using the MID (2);
- measuring data concerning a position and orientation of at least one object of the ARS;
- transforming the image taken by the MID (2) from a coordinate system of the image taken by the MID (2) ("CS_{image}") to a coordinate system of the OHMD (1) ("CS_{OHMD}") using the measured data; and
- displaying the transformed image in a position, orientation and scale that corresponds to the perspective of a position and orientation of the OHMD (1) ("PO_{OHMD}") in quasi-real-time on the OHMD (1).

12. The method of claim 11, wherein said at least one object comprises the MID 2 and/or the OHMD 1.

13. The method of claim 11 or 12, comprising measuring data concerning a spatial acceleration and a rotation rate of at least one object, and
wherein the step of transforming the image taken by the MID (2) from the CS_{image} to the CS_{OHMD} is performed using an estimate of the position and orientation of the at least one object that is calculated using
o the measured data concerning a spatial acceleration and a rotation rate of the at least one object; and
o an earlier determined position and orientation of the at least one object;
and wherein the at least one object preferably comprises the MID 2.

14. The method of one of claims 11 to 13, comprising switching from
o a first way of performing the step of transforming the image taken by the MID (2) from the CS_{image} to the CS_{OHMD} to
o a second way of performing the step of transforming the image taken by the MID (2) from the CS_{image} to the CS_{OHMD}
upon the occurrence of a triggering event;
and wherein the occurrence of the triggering event preferably comprises that a measurement that is used in the first way is deemed unreliable.

15. The method of claim 13 and claim 14, wherein the second way comprises the steps of claim 13;
and wherein the first way preferably does not comprise the steps of claim 13.
